# EUROPEAN PATENT APPLICATION

(11) **EP 3 210 616 A1**
(43) Date of publication of application: **30.08.2017**
(21) Application number: 15851823.3
(22) Date of filing: 23.10.2015
(51) Int. Cl.: A61K 38/16, A61K 39/395, A61P 3/10, A61P 1/18, A61P 37/00

(54) **USE OF MACROPHAGE INFLAMMATORY PROTEIN-1 (MIP-1 ) INHIBITOR TO PROTECT PANCREAS AND PREVENT BLOOD SUGAR FROM RISING**

(30) Priority: 24.10.2014 US 201462068475 P
(71) Applicant: Farmar Licensing Co., Ltd., Taichung City (TW); Chen, Jaw-Wen, Taipei City (TW)
(72) Inventor: CHEN, Jaw-Wen, Taipei (TW); CHANG, Ting-Ting, Taoyuan City Taoyuan County (TW)
(74) Representative: Viering, Jentschura & Partner mbB Patent- und Rechtsanwälte
(86) International application number: PCT/CN2015/092770
(87) International publication number: WO 2016/062282

(57) **Abstract**

The present invention relates to a use of a macrophage inflammatory protein-1β (MIP-1β) inhibitor to protect the pancreas and prevent blood sugar from rising. The present invention relates to a use of a macrophage inflammatory protein-1β (MIP-1β inhibitor to protect the pancreas, and to prevent blood sugar from rising in a diabetic subject.

## Description

### CROSS-REFERENCE TO RELATED APPLICATION

This application claims priority to U.S. Provisional Application No. 62/068475, filed on October 24, 2014, the entire content of which is hereby incorporated by reference herein in its entirety.

### BACKGROUND OF THE INVENTION

### Technical Field of the Invention

The present invention relates to a use of macrophage inflammatory protein-1β (MIP-1β inhibitor for protecting pancreas. Especially, the present invention relates to a use of MIP-1β inhibitor for maintaining the secretion of insulin and preventing the disordered elevation of blood sugar level in diabetic patients.

### Background

In recent years, the number of clinical diabetes in both the world or Asian countries, especially mainland China, Taiwan, Japan and India are increasing year by year. In Taiwan, the number of people suffering from type 2 diabetes is close to 10% of the total population, and still increasing. Therefore, there is an urgent need for the development of diabetes drugs. It is known that elevated blood glucose in diabetes is mainly caused by increased insulin resistance (in type 2 diabetes) and inflammation of pancreatic islet cells (in type 1 and type 2 diabetes), so that the insulin secretion of pancreas is insufficient to reduce the ingested and the manufactured sugar in the body.

Currently, the blood glucose control in clinical diabetes mellitus is mainly focused on the mechanisum of stimulating pancreas to secrete insulin (in the treatment of type 2 diabetes), reducing the insulin resistance in peripheral tissues (in the treatment of type 2 diabetes), reducing intestinal sugar intake and absorption (treatment of type 1 and type 2 diabetes) or increasing urinary sugar exclusion (treatment of type 1 and type 2 diabetes).

However, the pancreatic islet cells will eventually be destroyed by inflammation and resulting in a serious shortage of insulin secretion in both of the type 1 and type 2 diabetes, which must be treated by injected or inhaled supplementary insulin. This is mainly because that the currently avaiable or developing drugs can not directly protect the pancreas (especially, the pancreatic islet cells), reduce or restore its damage, and to maintain insulin secretion. Therefore, the present invention has been directed to develop a medicament for preventing hyperglycemia in diabetic patients by the way of protecting the pancreas of a diabetic pateint and improving the pathogenesis of pancreatic islet inflammation and destruction.

Macrophage inflammatory protein-1β (MIP-1β, also known as CCL4) is a member of the CC chemokine family that is first isolated from culture medium of LPS-activated macrophage (Lodi P.J., et al., Science 263:1762-1767, 1994). MIP-1βhas a molecular weight (MW) of 7.8 kDa. The protein structure of MIP-1β is constituted as a precursor of 92 amino acids. Mature secreted proteins as 69 amino acids are generated by peptidases that cleave hydrophobic signal peptides. The upregulation of MIP-1β was observed in DM and cardiovascular diseases (Tatara, Y., et al., J Mol Cell Cardiol 47:104-111 ,2009; Mirabelli-Badenier, M., et al., Thromb Haemost 105:409-420 ,2011).

MIP-1β mediate its biological effects by binding to cell surface CC chemokine receptors (CCRs), which belong to the G-protein-coupled receptor super-family. There are many macrophage inflammatory protein-1β receptors, in which the most well-known receptor of CCL4 is the fifth CC chemokine receptor (CCR5) and considered to have the main physiological and pathological effects. The anti-MIP-1β monoclonal antibody provided by the present invention can antagonize and regulate the function of CCR5. In an *animal model of experiment diabetes,* CCR5 expression in the pancreas could be associated with the development of insulitis and spontaneous type 1 DM (Cameron MJ, et al., J Immunol 165:1102-1110, 2000). CCR5 is also elevated in the superior cervical ganglion of type 2 diabetic rats. However, the data was not consistent in different animal models. It was reported in two previous studies that transient blockade of CCR5 with an anti-CCR5 mAb during 11-13 weeks of age or CCR5 deficiency significantly accelerates rather than prevents auto-immune type 1 diabetes in non-obese diabetic (NOD) mice (Gonzalez P, et al., Genes Immun 2:191-195, 2001; Simeoni E, et al. Eur Heart J 25:1438-1446, 2004).

Recent findings demonstrated that plasma macrophage inflammatory protein-1β values showed a positive correlation with the diabetes mellitus and the cardiovascular diseases. Thus, we develop a new treatment strategy that protects the pancreas from inflammation by inhibiting the function of macrophage inflammatory protein (MIP)-1β and further maintains insulin secretion, and thereby controls blood sugar.

### SUMMARY OF INVENTION

The present invention has been found on the above purposes that direct inhibition of macrophage inflammatory protein-1β such as through the monoclonal antibody and other methods, can produce effects of protecting the pancreas function, maintaining insulin secretion, and sopressing the continuous blood glucose level rising in the type 1 and type 2 diabetic animal models.

Accordingly, in one aspect, the present invention features a use of a macrophage inflammatory protein-1β (MIP-1β inhibitor to prepare a pharmarceutical composition for protecting the function of pancreas in a diabetic subject, wherein the protection of pancreas function comprises preventing with pancreatic island cell damage in the diabetic subject. In certain embodiments, the pharmarceutical composition is used to maintain insulin secretion in the diabetic subject. In other embodiments, the pharmarceutical composition is used to prevent the elevation of blood sugar in the diabetic subject.

In certain embodiments of the present invention, the MIP-1β inhibitor is a compound capable of decreasing or inhibiting the biological activity of MIP-1β. In one embodiment, the said MIP-1β inhibitor is a MIP-1β-specific ligand, such as an anti-MIP-1β antibody or an antagonist for MIP-1β.

In certain embodiments of the present invention, the anti-MIP-1β antibody is a polyclonal or monoclonal antibody against MIP-1β. In one embodiment, the anti-MIP-1β antibody is a monoclonal antibody, or a protein moiety thereof, having a binding site with binding specificity for a fragment of MIP-1β. In other embodiments, the fragment of MIP-1β comprises an amino acid sequence of 46SFVMDYYET54 (SEQ ID NO:1) or 62AVVFLTKRGRQIC74 (SEQ ID NO:2).

### BREIF DESCRIPTION OF THE DRAWINGS

Figure 1 shows the protective effects of MIP-1β inhibitor on pancreas in STZ-induced type 1 diabetic mice. The activity of macrophage inflammatory protein (MIP) -1β is inhibited by monoclonal antibody (mAb), which effectively increases the serum insulin levels (n=9).
Figure 2 shows the results of streptozotocin (STZ)-induced inflammation in pancreatic tissue of the type 1 diabetic animal model observed by section staining. The red fluorescence indicates insulin expression in islet cells.
Figure 3 shows the serum levels of MIP-1β in STZ-induced diabetic mice (n=6).
Figure 4 shows the inflammatory states in the pancreatic tissue of type 2 diabetic animal model with hyperinsulinaemia and hyperglycaemia observed by histochemical staining. The red fluorescence indicates insulin expression in islet cells.
Figure 5 shows the results of Western blot and statistical analysis of IL-6 and IL-8 in pancreas of mice (n=3; 3B). #P<0.05, ##P<0.01 compared with control mice. *P<0.05, **P<0.01 compared with untreated DM mice.
Figure 6A shows the concentration of MIP-1β in the supernatants of islet cell (NIT-1 cell) culture medium treated with different doses of STZ. The MIP-1β level was increased with the proliferation of NIT-1 cells. Figure 6A shows the cytotoxicity of different doses of STZ (STZ 1.5, STZ 3) to NIT-1 cells evaluated by MTT assay (n=3). The decreased NIT-1 cell proliferation is restored by the treatment of monoclonal antibody (mAb), which decreases the damages cause by STZ.
Figure 7 shows the insulin levels in the supernatants of NIT-1 cells (n=6). #P<0.05, ##P<0.01 compared with control group of NIT-1 cell. *P<0.05, **P<0.01 compared with the same STZ concentration treated NIT-1 cell group.

### DETAILED DESCRIPTION OF THE INVENTION

As used herein, "MIP-1β-inhibitor" refers to a compound that decreases the level of MIP-1β protein and/or decreases at least one activity of MIP-1β protein. In an exemplary embodiment, a MIP-1β-inhibiting compound may decrease at least one biological activity of a MIP-1β protein by at least about 10%, 25%, 50%, 75%, 100%, or more.

In certain embodiments, methods for reducing, preventing or treating diseases or disorders using a MIP-1β-modulating compound may also comprise decreasing the protein level of a MIP-1β, or homologs thereof. Decreasing MIP-1β protein level can be achieved according to methods known in the art. For example, a siRNA, an antisense nucleic acid, or a ribozyme targeted to the MIP-1β can be expressed in or be transfected into the cell. Alternatively, agents that inhibit transcription can be used. Methods for modulating MIP-1β protein levels also include methods for modulating the transcription of genes encoding MIP-1β, methods for destabilizing the corresponding mRNAs, and other methods known in the art.

In other embodiments, the MIP-1β-inhibitor directly or indirectly decreases or inhibits the activity of MIP-1β protein by binding to MIP-1β protein, and thereby to protect pancreas and prevent blood sugar from rising. For instance, according to some embodiments of the present invention, methods for inhibiting the activity of MIP-1β protein in a subject may use an anti-MIP-1β antibody to compete with the MIP-1β protein for binding to its receptor on cell surface. The term "antibody" herein is used in the broadest sense and specifically includes full-length monoclonal antibodies, polyclonal antibodies, multispecific antibodies (e.g., bispecific antibodies), and antibody fragments, as long as they exhibit the desired biological activity.

As used herein, the term "antibody" means an immunoglobulin molecule or a fragment of an immunoglobulin molecule having the ability to specifically bind to a particular antigen. An "antibody fragment" comprises a portion of a full-length antibody, preferably antigen-binding or variable regions thereof. Examples of antibody fragments include Fab, Fab', F(ab)₂, F(ab')₂, F(ab)₃, Fv (typically the VL and VH domains of a single arm of an antibody), single-chain Fv (scFv), dsFv, Fd fragments (typically the VH and CH1 domain), and dAb (typically a VH domain) fragments; VH, VL, and VhH domains; minibodies, diabodies, triabodies, tetrabodies, and kappa bodies (see, e.g., Ill et al., Protein Eng 1997;10: 949-57); camel IgG; and multispecific antibody fragments formed from antibody fragments, and one or more isolated CDRs or a functional paratope, where isolated CDRs or antigen-binding residues or polypeptides can be associated or linked together so as to form a functional antibody fragment.

In certain embodiments, the MIP-1β-inhibitor is a monoclonal antibody specifically binding to the MIP-1β protein. In one embodiment, the anti-MIP-1β monoclonal antibody has the binding specificity for a functional fragment of MIP-1β protein structure. According to some embodiments of present invention, the MIP-1β-inhibitor, such as a monoclonal antibody, binds to the antigen determinant fragment of MIP-1β protein comprising an amino acid sequence of 46-54: SFVMDYYET (SEQ ID NO:1) or 62∼74: AVVFLTKRGRQIC (SEQ ID NO:2).

In some embodiments of the invention, the monoclonal antibody is a humanized antibody or a human antibody.

Pharmaceutical compositions for use in accordance with the present invention may be formulated in a conventional manner using one or more physiologically acceptable carriers comprising excipients and auxiliaries which facilitate processing of the active compounds into preparations which can be used pharmaceutically. Proper formulations, including (but not limited to) oral compositions such as tablets, capsules, powders and the like, parenteral compositions such as aqueous solutions for subcutaneous, intramuscular or intraperitoneal injection, and lyophilized powders combined with a physiological buffer solution just before administration, are formulated depending upon the chosen route of administration.

The other characteristics and advantages of the present invention will be further illustrated and described in the following examples. The examples described herein are using for illustrations, not for limitations of the invention.

### Example

### Example 1. MIP-1β inhibition protects pancreas and prevents blood sugar from rising in Streptozotocin (STZ)-induced diabetic mice

The blood sugar concentrations were followed during *in vivo* experiment periods. The blood sugar levels of STZ-induced diabetic mice were not only increased in with or without the hindlimb ischemia surgery groups but in the MIP-1β neutralizing antibody injection for 2 weeks group. However, the blood sugar level was maintained in the MIP-1β neutralizing antibody injection for 4 weeks group no matter with or without the hindlimb ischemia surgery **(Table 1).**

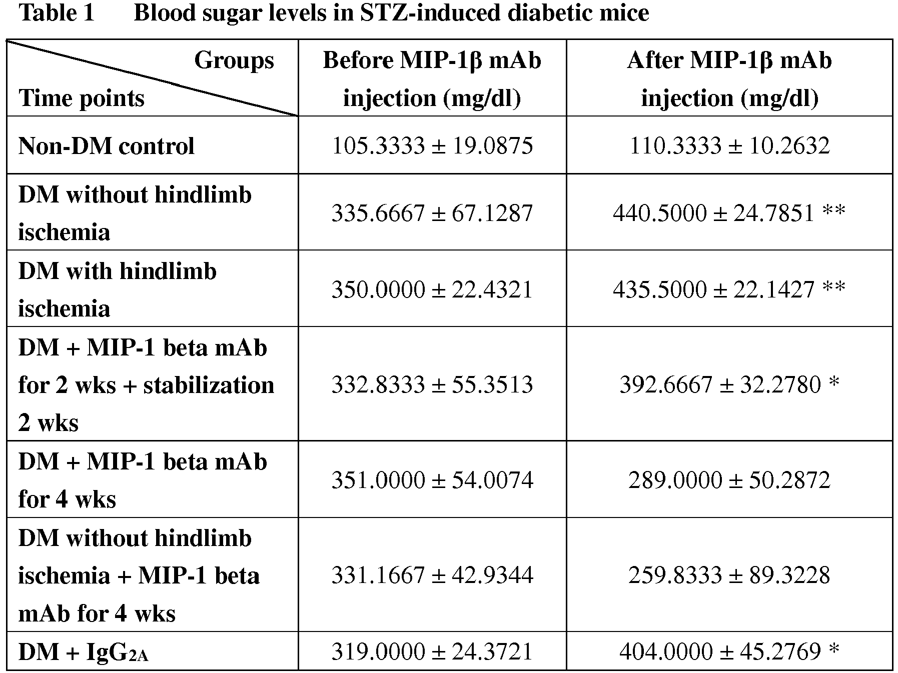

Values are presented as mean ± SD (n=6∼8 in each group). **P<0.05*, ***P<0.01* compared with the same group of blood sugar level before MIP-1β mAb injection.

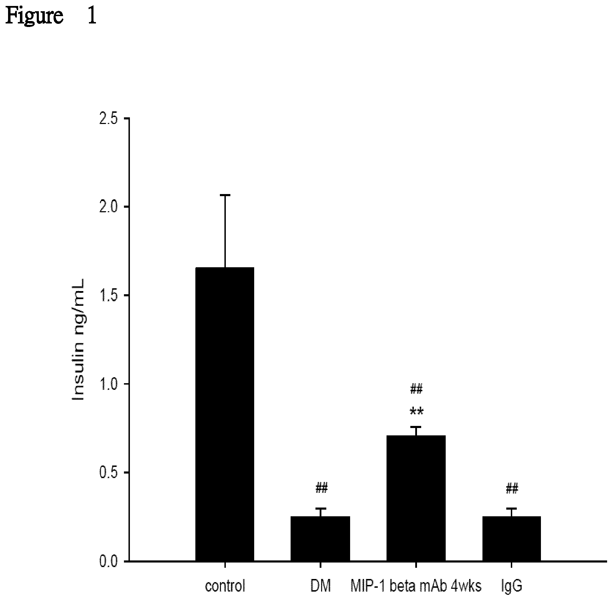

As showed in **Figure 1****,** serum concentrations of insulin were significantly decreased in STZ-induced diabetic mice compared with control mice. And, insulin levels were increased after MIP-1β neutralizing antibody injection for 4 weeks compared with untreated diabetic mice.

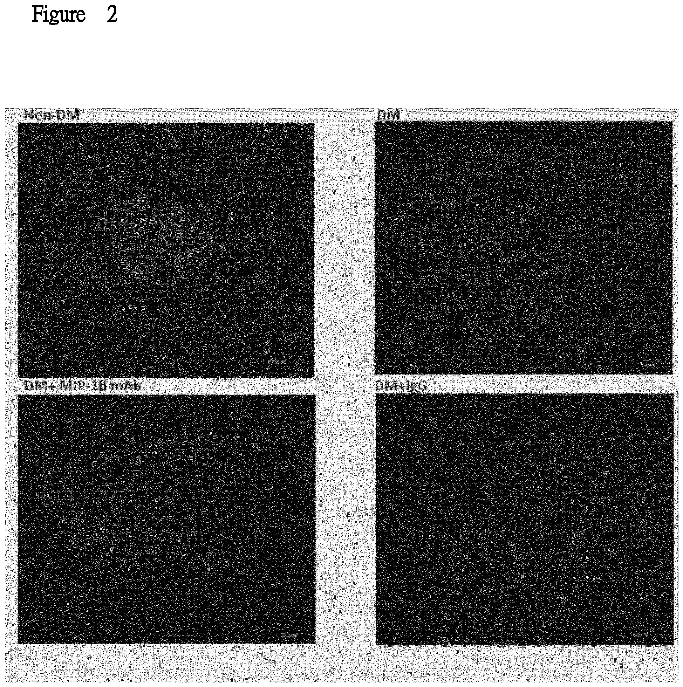

Additionally, the inflammatory state in pancreas tissue was observed by histochemical staining with insulin expression. As showed in **Figure 2****,** MIP-1β inhibition groups had more insulin expressions (by the islet cell having red fluorescence) in pancreas than untreated diabetic mice group. These results indicate that the number of pancreatic islets was significantly reduced due to inflammation damages in diabetic animals with high blood glucose levels (the DM group). On the contrary, the diabetic animals injected with a monoclonal antibody (mAb) against MIP-1β (the DM + mAb group) can prevent pancreatic island cells from the damages by inflammation, further partially maintain the morphology of pancreatic island and achieve the effects of protecting the pancreas.

### Example 2. MIP-1β inhibition protects pancreas and prevents blood sugar from rising in Leprdb/db type 2 diabetic mice

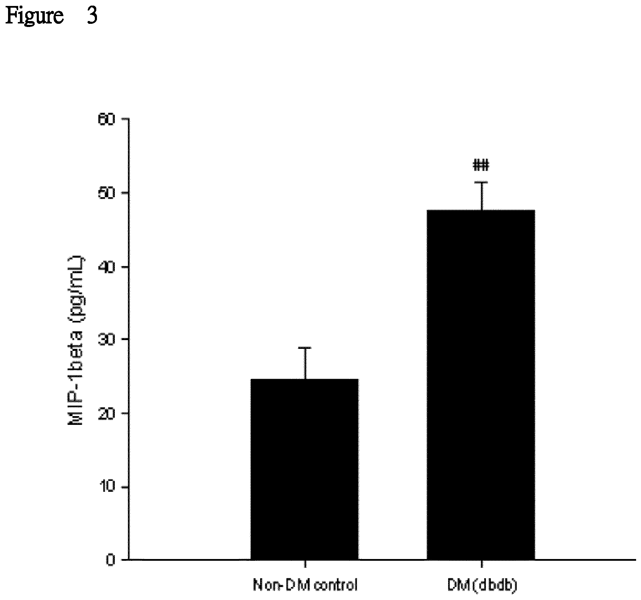

As the results showed in Figure 3, MIP-1β levels were elevated in high fat diet-induced Leprdb/db type 2 diabetic mice (produced by autosomal recessive mutation in the leptin receptor), compared to the normal control mice. Injection of an anti-MIP-1β monoclonal antibody can effectively control the blood glucose level, which does not continue to increase, in diabetic animals with high blood glucose level, no matter with or without the hindlimb ischemia surgery (OP) (Table 2).

**Table 2: Blood sugar levels in Leprdb/db diabetic mice**

| | **Before MIP-1β mAb injection (mg/dl)** | **After MIP-1β mAb injection (mg/dl)** |
|---|---|---|
| **non-DM control** | 154.6000±10.6442 | 157.4000±6.1074 |
| **DM without op** | 327.8000±43.5167 | 641.2000±51.178** |
| | N=5 | |
| **DM with op** | 325.3333±57.0322 | 656.3333±39.6669** |
| **DM + MIP-1 beta mAb for 2 wks + stabilization 2 wks** | 321.0000±55.0963 | 473.6667±58.5377** |
| **DM + MIP-1 beta mAb for 4 wks** | 342.6667±58.0299 | 377.0000±33.5857 |
| **DM without op+ MIP-1 beta mAb for 2 wks + stabilization 2 wks** | 287.6000±25.9191 | 480.8000±112.0299** |
| | N=5 | |
| **DM without op+ MIP-1 beta mAb for 4 wks** | 319.6000±92.7324 | 370.4000±84.2929 |
| | N=5 | |
| **DM + IgG_{2A}** | 363.2500±82.2086 | 629.7500 ±75.5177** |

| | | |
|---|---|---|
| Values are presented as mean ± SD (n=4∼6 in each group). *P<0.05, **P<0.01 compared with the same group of blood sugar level before MIP-1β mAb injection. | | |

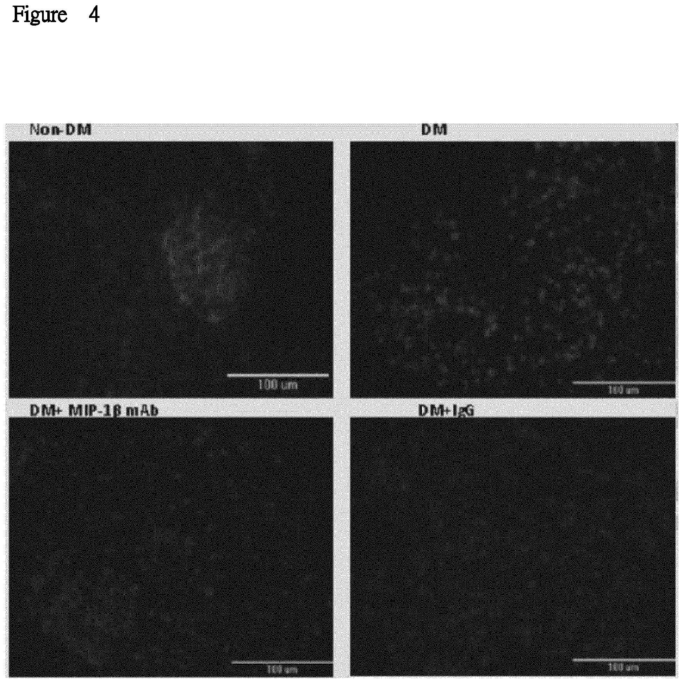

Furthermore, the inflammation in the pancreatic tissue of Leprdb/db type 2 diabetic mice model (which are hyperphagic, obese,hyperinsulinaemic and hyperglycaemic) was observed by histochemical staining. The red fluorescence presents the level of insulin expression for indicating islet cells. As showed in Figure 4, the islet cells of diabetic mice were severely damaged as losing red fluorescence. However, the morphology of islet cells of Leprdb/db type 2 diabetic mice with high blood glucose level was partially restored after the injection of MIP-1β monoclonal antibody (mAb). These results suggest that administration of MIP-1β neutralizing monoclonal antibody provides protective effects on pancreas to prevent islet cells from damage and maintain the normal function of insulin secretion in the pancreas.

These findings implied the possible protection of pancreas from MIP-1β inhibition in diabetic animals.

### Example 3. MIP-1β inhibitor protects islet cells from inflammation in Streptozotocin (STZ)-induced damage cell model

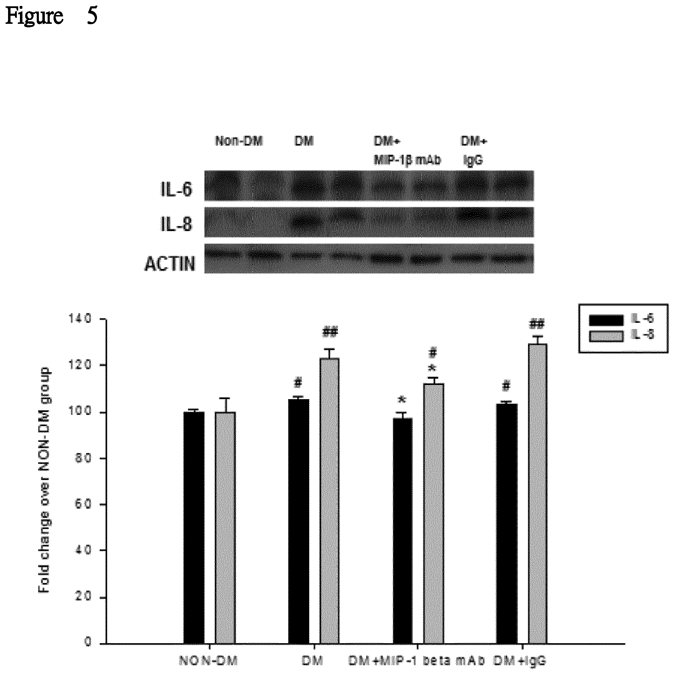

For further understanding the mechanism of the protective effects on pancreas by MIP-1β inhibitor, we evaluated the level of inflammatory proteins in pancreas by Western blotting. As showed in **Figure 5****,** the levels of interleukin 6 (IL-6) and interleukin 8 (IL-8) were both decreased in the MIP-1β neutralizing monoclonal antibody treated group (DM + MIP-1 beta mAb group). The results indicate that injection of an anti-MIP-1β monoclonal antibody effectively inhibit the expression of inflammatory proteins, such as IL-6 and IL-8, in pancreas (especially, in islet). Thus, inhibiting the function of MIP-1β protein will protect islet cells from the inflammation response induced by the STZ-treatment.

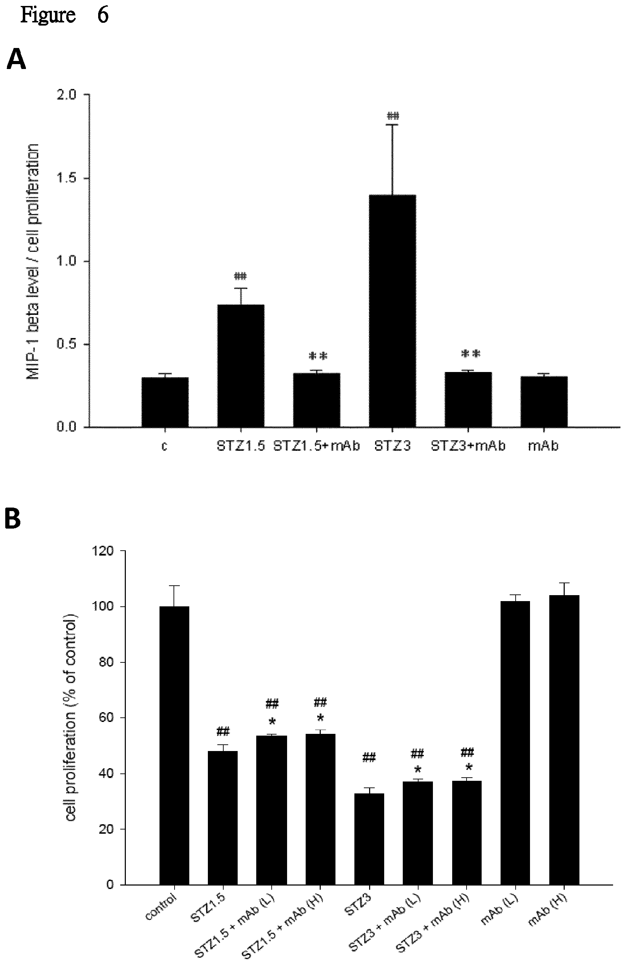

In the *in vitro* experiment, we added STZ to mimic the *in vivo* conditions in our previous experiments. NIT-1 (a pancreatic beta-cell line established from transgenic nod/It mice) cells were seeded in 96-well plates at a cell concentration of 1 × 10⁵ cells per well and were pre-incubated overnight. After pre-incubation, NIT-1 cells were exposed to STZ (0, 0.75, 1.5, 3, 6 mM) for 24 hours. Cytotoxicity of STZ on the NIT-1 cells was determined using MTT assay. Also, NIT-1 cells were treated with STZ for 24 hours and then with or without MIP-1β antibody (R&D system) at low dose (0.3 µg/ml) or high dose (30 µg/ml) for 4 hours. NIT-1 cell proliferation was evaluated by MTT assay. The results are showed in **Figure 6****.**

As the results of NIT-1 cell research showed in Figure 6A, streptozotocin (STZ 1.5, STZ 3) stinulated the islet cells (NIT-1 cells) to produce Macrophage inflammatory protein (MIP)-1β, which resulted in the increased concentration of MIP-1β in the culture medium with the incresing dose of streptozotocin. The increase in macrophage inflammatory protein-1β production was inhibited by the administration of anti-MIP monoclonal antibody (mAb).

As showed in **Figure 6B****,** NIT-1 cell viability was significantly decreased after the streptozotocin (STZ 1.5, STZ 3) treatments. The cytotoxicity of STZ on the islet cells (NIT-1 cells) was increased with the dose of STZ. The antagonistic effect of monoclonal antibody (mAb) against the produced MIP-1β can directly and effectively improve the inhibition of islet cells (NIT-1) proliferation and cell damages induced by streptozotocin (STZ 1.5, STZ3). Therefore, inhibition of macrophage inflammatory protein (MIP)-1β can directly protect islet cells and reduce the damage of streptozotocin to islet cells.

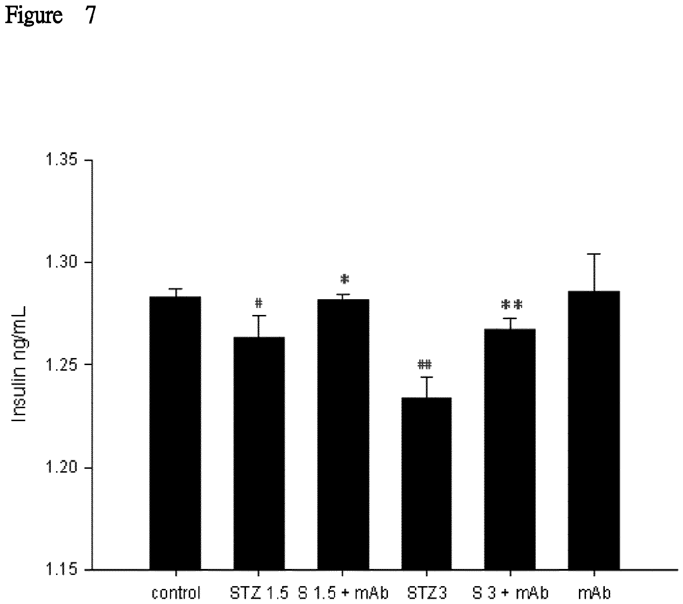

The results in **Figure 7** showed that the insulin secretion in NIT-1 cell supernatants were decreased after 1.5 or 3 mM STZ treatment, and the decrease is dose-dependent. The inhibition of insulin secretion can be reversed by the treatment of MIP-1β neutralizing monoclonal antibody (mAb). These in vitro data confirmed that MIP-1β inhibition was benefit to protect islet directly, reduce the damages induced by STZ, and recover the decreased insulin secretion.

As one embodiment of inhibitory agent for MIP-1β, the MIP-1β neutralizing antibody exhibits effects on type 1 and type 2 diabetic animals, including to protect pancreas from inflammation; maintain the blood sugar levels after MIP-1β neutralizing antibody treatments; increase insulin levels; and decrease IL-6/IL-8 expressions in pancreas, indicating the protection role of MIP-1β inhibition in pancreas function.

ÐòÁÐ±í

<110>  •¨Âê¿Æ¼¼¹ËÎÊ¹É•ÝÓÐÏÞ¹«Ë¾
       ³ÂÕØÎÄ

<120>  ¾ÞÊÉÏ¸°û•¢Ñ×µ°°×-1¦Â(MIP-1¦Â)ÒÖÖÆ¼ÁÓÃÒÔ±£»¤ÒÈÔà¼°•ÀÖ¹Ñª¹ÇÉý¸ßµÄÓÃÍ¾

<130>

<160>  2

<170>  PatentIn 3.5°æ

<210>  1
<211>  9
<212>  PRT
<213>  Ð¡Êó (Mus musculus)

<400>  1
Ser Phe Val Met Asp Tyr Tyr Glu Thr
1               5

<210>  2
<211>  13
<212>  PRT
<213>  Ð¡Êó (Mus musculus)

<400>  2
Ala Val Val Phe Leu Thr Lys Arg Gly Arg Gln Ile Cys
1               5                   10

## Claims

1. A use of a macrophage inflammatory protein-1β (MIP-1β inhibitor to prepare a pharmarceutical composition for protecting the function of pancreas in a diabetic subject, wherein the protection of pancreas function comprises preventing with islet cell damage in the diabetic subject.

2. The use of claim 1, wherein the pharmarceutical composition is used to maintain insulin secretion in the diabetic subject.

3. The use of claim 1, wherein the pharmarceutical composition is used to prevent the elevation of blood sugar in the diabetic subject.

4. The use of claim 1, wherein the macrophage inflammatory protein-1β inhibitor is a compound capable of decreasing or inhibiting the biological activity of MIP-1β.

5. The use of claim 1 or 4, wherein the macrophage inflammatory protein-1β (MIP-1β) inhibitor is a ligand compound with binding specificity for MIP-1β.

6. The use of claim 5, wherein the MIP-1β inhibitor is an antibody against MIP-1β.

7. The use of claim 6, wherein the antibody is a monoclonal antibody with binding specificity for MIP-1β or a MIP-1β fragment.

8. The use of claim 7, wherein the monoclonal antibody comprises a protein moiety that has a binding site with a fragment of MIP-1β.

9. The use of claim 7 or 8, the monoclonal antibody comprises a binding site for a fragment of MIP-1β comprising an amino acid sequence of SFVMDYYET (SEQ ID NO:1).

10. The use of claim 7 or 8, the monoclonal antibody comprises a binding site for a fragment of MIP-1β comprising an amino acid sequence of AVVFLTKRGRQIC (SEQ ID NO:2).
